(19) Europäisches Patentamt
European Patent Office
Office européen des brevets

(11) EP 2 957 553 A1

(12) EUROPEAN PATENT APPLICATION

(43) Date of publication:
**23.12.2015 Bulletin 2015/52**

(21) Application number: **15172162.8**

(22) Date of filing: **15.06.2015**

(51) Int Cl.:
***C07C 31/135*** *(2006.01)* ***A61K 31/40*** *(2006.01)*
***A61P 11/08*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **16.06.2014 TR 201406982**
**17.06.2014 TR 201407012**
**17.06.2014 TR 201407009**
**17.06.2014 TR 201407010**

(71) Applicant: **Arven Ilac Sanayi Ve Ticaret A.S.**
**Istanbul 34460 (TR)**

(72) Inventors:
- **TÜRKYILMAZ, Ali**
**34460 Istanbul (TR)**
- **CELIK, Devrim M.**
**34460 Istanbul (TR)**
- **AKDAS, Özlem M.**
**34460 Istanbul (TR)**

(74) Representative: **Sevinç, Erkan**
**Istanbul Patent A.S.**
**Plaza-33, Büyükdere Cad. No: 33/16**
**Sisli**
**34381 Istanbul (TR)**

# (54) PHARMACEUTICAL FORMULATIONS OF VILANTEROL

(57) The present invention relates to a pharmaceutical formulation for inhalation comprising vilanterol as an active agent, a carrier and magnesium stearate in the treatment and prophylaxis of respiratory diseases such as asthma, chronic obstructive pulmonary disease (COPD) as well as to a process for producing this formulation.

EP 2 957 553 A1

## Description

### Field of the invention

The present invention relates to a pharmaceutical formulation for inhalation comprising vilanterol as an active agent, a carrier and magnesium stearate in the treatment and prophylaxis of respiratory diseases such as asthma, chronic obstructive pulmonary disease (COPD) as well as to a process for producing this formulation.

### Background of the invention

$\beta_2$-adrenoceptor agonists are widely used in the treatment and/or prophylaxis of respiratory diseases associated with airflow obstruction such as asthma, chronic obstructive pulmonary disease (COPD). They provide rapid and effective symptom relief principally by opposing the bronchoconstriction induced by excitatory airway mediators. Therefore, $\beta_2$-adrenoceptor agonists improve lung function, symptoms of breathlessness and exercise limitation, health-related quality of life, and may also reduce the rate of exacerbations.

There are two types of $\beta_2$-adrenoceptor agonists: long-acting $\beta_2$-adrenoceptor agonist (LABA) and short-acting $\beta_2$-adrenoceptor agonist (SABA). Short-acting $\beta_2$- adrenoceptor agonists are used only as relief medication, whereas long-acting $\beta_2$- adrenoceptor agonists are used on a daily basis to improve lung function.

A LABA with a 24-hour duration of action could provide improvements in efficacy, compared with twice-daily LABAs, and the once-daily dosing regimen could help improve compliance. An important step in simplifying asthma and COPD management and improving compliance with prescribed therapy is to reduce the dose frequency to the minimum necessary to maintain disease control. Therefore, once-daily dose administration is an important strategy to improve compliance.

Vilanterol is a LABA with a 24-hour duration of action that is used for the preparation of a medicament in the prophylaxis and treatment of respiratory diseases such as asthma, chronic obstructive pulmonary diseases (COPD), respiratory tract infection and upper respiratory tract disease. It is also known with the chemical name of 4-{(1R)-2-[(6-{2-[(2,6-dichlorobenzyl)oxy]ethoxy}hexyl)amino]-1-hydroxyethyl}-2-(hydroxymethyl) phenol of fomula given below:

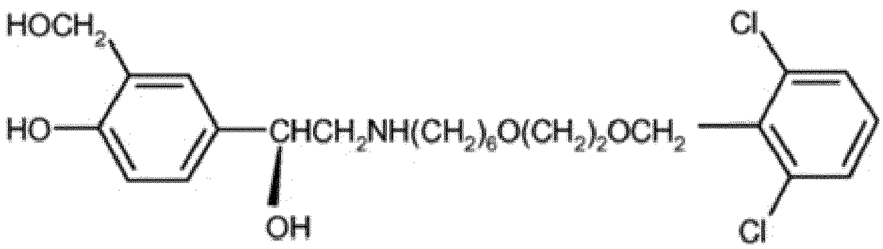

**Compound (I)**

The compound (I) and pharmaceutically acceptable salts thereof, in particular the acetate, triphenylacetate, a-phenylcinnamate, 1 -naphthoate and (R)-mandelate salts, are specifically described in WO03/024439A1 as well as the preparation method of the compound I.

$\beta_2$-adrenoceptor agonists used in the treatment of respiratory diseases are administered through various routes (e.g. inhalation, oral, parenteral). However, the preferred route of administration of these drugs is inhalation, since they are directly delivered to the affected sites (airways) in high doses via this route, have a short onset time, and they lack or have minimal systemic side effects. The pharmaceutical formulations comprising said drugs are administered via nebulizers, pressurized metered dose inhalers (pMDIs), and dry powder inhalers (DPIs). In nebulizers and pMDIs, drugs are dispersed in water or propellant-solvent mixtures, and administered as aerosolized droplets of the dispersed systems. But in DPIs, drugs are administered as a powder after formulating them with inert carriers, including lactose, glucose, and mannitol. Compared to other pulmonary drug delivery systems, DPIs offer several advantages, including enhanced drug stability, greater accuracy in dosing, elimination of hand-to-mouth coordination, breath-actuated delivery, and consequently, an overall improvement in patient compliance.

Dry powder formulations, that are suitable to be used via DPI, must fulfil a number of demands. With the aim of fulfilling these demands, it would be highly advantageous to provide a formulation exhibiting good uniformity of distribution of the active ingredient, small drug dosage variation (in other words, adequate accuracy of the delivered doses), good flowability, adequate physical stability in the device before use, good aerosolisation performance in terms of emitted dose and fine particle fraction (FPF).

The effective inhalation performance of dry powder products is dependent on the drug formulation and the inhaler device. Dry powder formulations are usually prepared by mixing the micronized drug particles with larger carrier particles. Drug deposition in the lung is mainly controlled by its mass median aerodynamic diameter. Particles larger than 5 μm are mostly trapped by oropharyngeal deposition and incapable of reaching the lungs while smaller than 1 μm are mostly exhaled without deposition. Particles with mass median aerodynamic diameters between 1 μm and 5 μm are expected to efficiently deposit in the lung periphery. However, microfine powder particles have high surface energy and thus they are highly adhesive and cohesive. It is well known that the surface of the carrier particles is, indeed, not smooth but has asperities and clefts, which are high energy sites on which the highly adhesive and cohesive active particles are preferably attracted to and adhere more strongly. These forces which occur between the two ingredients in the powder mixtures turn out to be too high thus preventing the separation of the micronised drug particles from the surface of the carrier particles during inhalation. Therefore, the magnitude of adhesion and cohesion forces is very important to produce a stable formulation that is homogenous mixture with proper content uniformity and with no powder segregation during the processing, storing and transportation, but allows for easy separation between drug and carrier particles during inhalation.

On the other hand, strong adhesion and cohesion forces lead to poor flowability and to powder aggregation. The poor flowability is also detrimental to the fine particle fraction (respirable fraction) of the delivered dose being the active particles unable to leave the inhaler and remaining adhered to the interior of the inhaler or leaving the inhaler as large agglomerates; agglomerated particles, in turn, cannot reach the bronchiolar and alveolar sites of the lungs. The uncertainty as to the extent of agglomeration of the particles between each actuation of the inhaler and also between inhalers and different batches of particles, leads to poor dose reproducibility as well.

Additionally, the poor flowability makes the powder to be difficult to handle and impairs the mechanical filling of the powder into medicament carriers (e.g. capsules, blisters, resevoirs, etc) with high accuracy and in correct dosage.

The patient compliance is very essential for the successful management of the respiratory diseases, especially asthma and COPD. For improvement in patient compliance, the important step is to reduce the dose frequency to the minimum necessary to maintain the treatment and this results in simplifying the management of respiratory diseases, especially asthma and COPD. Therefore, the once-daily dose administration is an important strategy to improve patient compliance.

In dry powder formulations for inhalation, it is necessary to deliver the drug with 24-hour duration of action to the lungs in efficient amount for the treatment to guarantee the maintenance the effect of the drug during 24-hour duration for success of the once-daily administration of said formulation.

Vilanterol is a LABA with a 24-hour duration of action that is used for the preparation of a medicament in the prophylaxis and treatment of respiratory diseases such as asthma, chronic obstructive pulmonary diseases (COPD), respiratory tract infection and upper respiratory tract disease. It is aimed in the present invention to provide a pharmaceutical formulation in dry powder form comprising vilanterol that overcomes the aforementioned drawbacks relating to the dry powders to deliver vilanterol in efficient amount in each inhalation to guarantee the maintenance the effect of vilanterol during 24-hour duration for the once-daily administration of said formulation.

## The Detailed Description of the Invention

The present invention relates to a dry powder formulation comprising vilanterol in which fine balance of the adhesive and cohesive forces between the drug and carrier particles is provided so as to ensure increase in the delivery efficiency of vilanterol to the lungs.

Dry powder formulations are generally formulated as a powder mixture of coarse carrier particles and micronized drug particles with mass median aerodynamic particle diameters of 1-5 μm. Carrier particles are used to improve drug particle flowability, thus improving dosing accuracy and minimizing the dose variability observed with drug formulations alone while making them easier to handle during manufacturing operations. With the use of carrier particles, drug particles are emitted from capsules and devices more readily, hence, the inhalation efficiency increases.

Usually no more than a few micrograms of a drug is used for the inhalation therapy, so it is very difficult to deliver the drug completely to the lung. Therefore, the carrier is used to enable bulk, which improves the handling, dispensing, and metering of the drug. Consequently, the carrier forms an important component of the formulation and any change in the properties of the carrier particles has the potential to alter the drug deposition profile. Therefore, the design of the carrier particle in terms of particle size, shape and surface properties is important for the aerosolization efficiency of dry powder formulations.

In order to get drug delivery into the lungs from a DPI formulation, the drug particles have to detach from surface of the carrier particles and penetrate into the lungs. The adhesive and cohesive forces between contiguous particle surfaces affect the detachment of the drug particles from surface of the carrier particles, and thus aerosolisation of respirable particles in dry powder formulation. The different surface properties of the carrier resulted in different adhesive forces between the drug and the carrier, which was reflected in the lung deposition results. During inhalation, the adhesive

forces that exist between drug and carrier particles have to be overcome in order to aerosolise drug particles. The magnitude of the attachment forces during inhalation relative to the adhesive forces in the mixture determines the obtained fine particle fraction (FPF). Consequently, optimising a dry powder inhalation system with respect to delivered fine particle dose requires careful balancing between both types of forces. The attachment forces have to be strong enough to maintain satisfactory blend homogeneity during handling, storage and transportation but weak enough to yield a high drug release from the carrier particles during inhalation.

It is know that the addition of low surface free energy materials such as magnesium stearate, leucine, lecithin to the carrier-based dry powder formulation increases the aerosolisation efficiencies of dry powder inhaler formulations, by decreasing the drug-excipient adhesion and thus facilitating the drug detachment upon device actuation. The magnesium stearate is preferably used, for this purpose, in the dry powder formulations. When magnesium stearate is used in the dry powder formulations, some of the active sites of carrier particles are occupied by magnesium stearate and the interparticulate forces (i.e. adhesive forces and cohesive forces) are balanced in the formulation, therefore not being too weak which would lead to the falling off the drug particles and forming agglomerates and not too strong which would not enable their detachment from the carrier surface. However, only some of the active sites are occupied by magnesium stearate, enabling some active sites to be occupied by the drug particles and therefore aerosolise upon actuation.

Accordingly, the main object of the present invention to provide dry powder formulations of vilanterol for inhalation in which the interparticulate forces between vilanterol particles and carrier particles are balanced for achieving an efficient inhalation of said formulation to guarantee the maintenance the effect of vilanterol during 24-hour duration for the once-daily administration of said formulation.

It has been surprisingly found that when the dry powder formulation for inhalation is formulated to comprise vilanterol or a pharmaceutically acceptable salt thereof, a pharmaceutically acceptable carrier and magnesium stearat wherein the quotient X is the ratio of the total amount of vilanterol (as free base) to the total amount of magnesium stearate and the quotient Y is the ratio of the volume median diameter of vilanterol or a pharmaceutically acceptable salt thereof to the volume median diameter of magnesium stearate, in which the ratio of the quotient X to the quotient Y is between 100:1 and 1:100, the adhesive and cohesive forces between vilanterol and the pharmaceutically acceptable carrier particles are well-balanced and the dry powder formulation that exhibits good uniformity of distribution of vilanterol, small drug dosage variation (in other words, adequate accuracy of the delivered doses), good flowability, adequate stability in the device before use, good aerosolisation performance in terms of emitted dose and fine particle fraction (FPF) is provided.

According to the present invention, more spesifically, the quotient Y is the ratio of the volume median diameter of vilanterol or a pharmaceutically acceptable salt thereof to the volume median diameter of the magnesium stearate whereas the quotient X is the ratio of the total amount of vilanterol to the total amount of the magnesium stearate. When the quotient X is calculated, the total amount of vilanterol calculated as free base is used.

$$X = \frac{\text{the total amount of vilanterol (as free base)}(\mu g)}{\text{the total amount of the magnesium stearate } (\mu g)}$$

$$Y = \frac{\text{the volume median diameter of vilanterol or a pharmaceutically acceptable salt } (\mu m)}{\text{the volume median diameter of the magnesium stearate } (\mu m)}$$

The carrier particles have a heterogeneous rough surface with clefts and asperities that enables a range of weak and strong adhesive interaction between the drug particles and the carrier surface and thus particle detachments occur over a wide range of energies. When the quotient X of the total amount of vilanterol (as free base) to the total amount of magnesium stearate and the quotient Y of the volume median diameter of vilanterol or a pharmaceutically acceptable salt thereof to the volume median diameter of magnesium stearate is present in a ratio within defined range in the dry powder formulation comprising vilanterol or a pharmaceutically acceptable salt, a synergistic effect is obtained in terms of interparticulate forces between vilanterol particles and carrier particles in comparison with the dry powder formulation of vilanterol or a pharmaceutically acceptable salt in which the ratio between the quotient X and the quotient Y does not satisfy defined provision. Addition of vilanterol and magnesium stearate with the provision that they satisfy the aforementioned ratio between the quotient X and the quotient Y, improves the aerodynamic performance of vilanterol in such a way that the number of free active sites with a wide range of energies on the pharmaceutically acceptable carrier surface was occupied by magnesium stearate particles and vilanterol particles is more effectively delivered.

In other words, this results in the interaction between vilanterol and the pharmaceutically acceptable carrier particles to be optimized to produce the stable formulation that is homogenous mixture with proper content uniformity and with no powder segregation during the processing, storing and transportation, but allows for easy separation between vilanterol and carrier particles during inhalation. The vilanterol particles are detached from the carrier surface easily in

dry powder formulations when exposed to an air flow of the patient during inhalation. Thus, the dry powder formulation of the present invention improves aerosolisation performance in terms of emitted dose and fine particle fraction (FPF) to be provided.

According to the present invention, "emitted dose (or delivered dose)" is the total amount of the active agent emitted from the inhaler device and hence available to the user. Fine particle fraction (FPF) is defined as the percentage of active agent (<5 μm in mass median aerodynamic diameter) which is deposited into respirable regions of the lung, divided by the total amount of active agent leaving the device. In order to achieve a good aerosolisation performance when dry powder formulations are developed, it is generally intended that the emitted dose is above 90% and the FPF is above 15%.

The volume median diameter ($Dv_{50}$ or $Dv_{0.5}$) is the median for a volume distribution in such a way that 50% of the volume of the particle diameter is less than the median and 50% of the volume of the particles diameter is more than the median.

The volume median diameter of the pharmaceutically acceptable carrier, the magnesium stearate and vilanterol or a pharmaceutically acceptable salt thereof contained in the dry powder formulation according to the present invention is preferably measured by means of a laser diffraction method. More specifically, the volume median diameter of the pharmaceutically acceptable carrier and the volume median diameter of the magnesium stearate are measured using a dry dispersion method using air as a dispensing agent on a "Malvern Mastersizer 2000 Particle Size Analyzer". The volume median diameter of vilanterol or a pharmaceutically acceptable salt thereof is measured using a dry dispersion or a liquid dispersion method, whichever is appropriate, making use of a suitable dispensing agent (air, water, solvent, etc) on a "Malvern Mastersizer 2000 Particle Size Analyzer".

In one embodiment of the present invention, the present invention provides a dry powder formulation for inhalation comprising vilanterol or a pharmaceutically acceptable salt thereof, a pharmaceutically acceptable carrier and magnesium stearat wherein the quotient X is the ratio of the total amount of vilanterol to the total amount of magnesium stearate and the quotient Y is the ratio of the volume median diameter of vilanterol to the volume median diameter of magnesium stearate, in which the ratio of the quotient X to the quotient Y is between 100:1 and 1:100, for example 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95; preferably between 75:1 and 1:100, for example 72:1, 69:1, 66:1, 63:1, 57:1, 54:1, 51:1, 48:1, 44:1, 42:1, 39:1, 36:1, 33:1, 29:1, 27:1, 24:1, 21:1, 18:1, 14:1, 12:1, 9:1, 6:1, 3:1, 1:3, 1:6, 1:9, 1:12, 1:16, 1:18, 1:21, 1:24, 1:27, 1:31, 1:33, 1:36, 1:39, 1:42, 1:44, 1:48, 1:51, 1:54, 1:57, 1:61, 1:63, 1:66, 1:69, 1:71; more preferably between 50:1 and 1:75, for example 49:1, 48:1, 47:1, 46:1, 45.5:1, 44:1, 43:1, 42.5:1, 41:1, 40:1, 39.5:1, 39:1, 38:1, 37:1, 36:1, 35:1, 34.5:1, 33.5:1, 32.5:1, 31.5:1, 29.5:1, 28.5:1, 27.5:1, 26.5:1, 25.5:1, 24.5:1, 23.5:1, 22.5:1, 21.5:1, 20.5:1, 19.5:1, 18.5:1, 17.5:1, 16.5:1, 15.5:1, 14.5:1, 13.5:1, 12.5:1, 11.5:1, 10.5:1, 9.5:1, 8.5:1, 7.5:1, 6.5:1, 5.5:1, 4.5:1, 3.5:1, 2.5:1, 1.5:1, 0.5:1, 1:0.5, 1:4, 1:5.5, 1:7, 1:8.5, 1:9.5, 1:10.5, 1:11, 1:12.5, 1:13, 1:14.5, 1:15.5, 1:17.5, 1:20.5, 1:21.5, 1:23, 1:24.5, 1:25.5, 1:26.5, 1:28, 1:29.5, 1:30.5, 1:31.5, 1:32.5, 1:33.5, 1:34.5, 1:35.5, 1:36.5, 1:37.5, 1:38.5, 1:39.5, 1:40.5, 1:41.5, 1:42.5, 1:43.5, 1:44.5, 1:45.5, 1:46.5, 1:47.5, 1:48.5, 1:49.5, 1:50.5, 1:51.5, 1:52.5, 1:53.5, 1:54.5, 1:55.5, 1:56.5, 1:57.5, 1:58.5, 1:59.5, 1:60.5, 1:61.5, 1:62.5, 1:63.5, 1:64.5, 1:65.5, 1:66.5, 1:67.5, 1:68.5, 1:69.5, 1:70.5, 1:71.5, 1:72.5, 1:73.5, 1:74.5; most preferably between 25:1 and 1:50, for example 24.4:1, 24.2:1, 23.8:1, 23.4:1, 22.8:1, 22.4:1, 21.8:1, 21.4:1, 20.8:1, 20.4:1, 19.8:1, 19.4:1, 18.8:1, 18.4:1, 17.8:1, 17.4:1, 16.8:1, 16.4:1, 15.8:1, 15.4:1, 14.8:1, 14.4:1, 13.8:1, 13.4:1, 12.8:1, 12.4:1, 11.8:1, 11.4:1, 10.8:1, 10.4:1, 9.8:1, 9.4:1, 8.8:1, 8.4:1, 7.8:1, 7.4:1, 6.8:1, 6.4:1, 5.8:1, 5.4:1, 4.8:1, 4.4:1, 3.8:1, 3.4:1, 2.8:1, 2.4:1, 2.2:1, 1.8:1, 1.6:1, 1.4:1, 1.2:1, 0.8:1, 0.4:1, 0.2:1, 1:1.4, 1:1.8, 1:2.2, 1:2.6, 1:3.2, 1:3.6, 1:3.8, 1:4.4, 1:4.8, 1:5.2, 1:5.6, 1:6.2, 1:6.6, 1:6.8, 1:7.2, 1:7.4, 1:7.6, 1:7.8, 1:8.2, 1:8.6, 1:8.8, 1:9.2, 1:9.6, 1:10.2, 1:10.4, 1:10.8, 1:11.2, 1:11.6, 1:11.8, 1:12.2, 1:12.4, 1:12.8, 1:13.2, 1:13.4, 1:13.6, 1:13.8, 1:14.2, 1:14.6, 1:15.2, 1:15.6, 1:15.8, 1:16.2, 1:16.4, 1:16.6, 1:16.8, 1:17.2, 1:17.4, 1:17.6, 1:17.8, 1:18.2, 1:18.6, 1:18.8, 1:19.2, 1:19.4, 1:19.8, 1:20.2, 1:20.4, 1:20.6, 1:20.8, 1:21.4, 1:21.8, 1:22.4, 1:22.8, 1:23.2, 1:23.6, 1:23.8, 1:24.2, 1:24.4, 1:24.8, 1:25.2, 1:25.4, 1:25.6, 1:25.8, 1:26.2, 1:26.4, 1:26.8, 1:27.2, 1:27.6, 1:27.8, 1:28.2, 1:28.4, 1:28.8, 1:29.2, 1:29.4, 1:29.8, 1:30.2, 1:30.4, 1:30.8, 1:31.2, 1:31.4, 1:31.8, 1:32.2, 1:32.4, 1:32.8, 1:33.2, 1:33.4, 1:33.8, 1:34.2, 1:34.4, 1:34.8, 1:35.2, 1:35.4, 1:35.8, 1:36.2, 1:36.4, 1:36.8, 1:37.2, 1:37.4, 1:37.8, 1:38.2, 1:38.4, 1:38.8, 1:39.2, 1:39.4, 1:39.8, 1:40.2, 1:40.4, 1:40.8, 1:41.2, 1:41.4, 1:41.8, 1:42.2, 1:42.4, 1:42.8, 1:43.2, 1:43.4, 1:43.8, 1:44.2, 1:44.4, 1:44.8, 1:45.2, 1:45.4, 1:45.8, 1:46.2, 1:46.4, 1:46.8, 1:47.2, 1:47.4, 1:47.8, 1:48.2, 1:48.4, 1:48.8, 1:49.2, 1:49.4, 1:49.8.

The dry powder formulation of the present invention has also a good flowability for inhaler filling and subsequently the fludisation of the formulation during device activation. This allows accurate metering of said dry powder formulation. Therefore, said formulation can be uniformly filled into blisters, capsules or reservoirs suitably used in dry powder inhalers, and thus, any dose inhaled by a patient from the respective blister, capsule, or reservoir during inhalation can be delivered with a high dose accuracy. Having said that, the dry powder formulation with good flow properties contributes to an almost complete discharge of the powder from the inhaler during inhalation.

Most of the amount of the dry powder formulations is consisted of carrier particle and the carrier particles are used to improve drug particle flowability, thus improving dosing accuracy and minimizing the dose variability observed

with drug formulations alone while making them easier to handle during manufacturing operations. With the use of carrier particles, drug particles are emitted from capsules and devices more readily, hence, the inhalation efficiency increases. Because of that, the selection of the carrier is very important for the aerosolisation performance of the dry powder formulation.

According to present invention, the pharmaceutically acceptable carrier used in the dry powder formulation that is selected from the group comprising lactose, mannitol, glucose, trehalose, cellobiose, sorbitol, maltitol or a combination of two or more of them. Lactose is preferably used as a pharmaceutically acceptable carrier in the dry powder formulation of the invention. In one embodiment of the present invention, lactose is anyhdrous lactose or lactose monohydrate.

According to the invention, "Pharmaceutical acceptable" refers to the properties and/or substances which are acceptable to the patient from a pharmacological-toxicological point of view and to the manufacturing pharmaceutical formulation.

The amount of the pharmaceutically acceptable carrier is much more than the total amount of active agent and magnesium stearate in the dry powder formulation of the present invention. Therefore, the particle size of the coarse carrier particles is also important for delivery performance of vilanterol in terms of fine particle fraction (FPF). The volume median diameter of the pharmaceutically acceptable carrier, preferably lactose, used in the dry powder formulation of the present invention, is between 30 μm and 250 μm, for example 35 μm, 40 μm, 45 μm, 50 μm, 55 μm, 60 μm, 65 μm, 70 μm, 75 μm, 80 μm, 85 μm, 90 μm, 95 μm, 100 μm, 105 μm, 110 μm, 115 μm, 120 μm, 125 μm, 130 μm, 135 μm, 140 μm, 145 μm, 150 μm, 155 μm, 160 μm, 165 μm, 170 μm, 175 μm, 180 μm, 185 μm, 190 μm, 195 μm, 200 μm, 205 μm, 210 μm, 215 μm, 220 μm, 225 μm, 230 μm, 235 μm, 240 μm, 245 μm; preferably between 40 μm and 225 μm, for example 43 μm, 48 μm, 57 μm, 64 μm, 76 μm, 82 μm, 93 μm, 106 μm, 119 μm, 121 μm, 133 μm, 142 μm, 151 μm, 165 μm, 173 μm, 186 μm, 192 μm, 203 μm, 207 μm, 211 μm, 216 μm, 218 μm, 222 μm; more preferably between 45 μm and 215μ m, for example 47 μm, 52 μm, 58 μm, 66 μm, 72 μm, 83 μm, 91 μm, 103 μm, 117 μm, 125 μm, 132 μm, 138 μm, 143 μm, 149 μm, 154 μm, 159 μm, 162 μm, 168 μm, 174 μm, 179 μm, 183 μm, 188 μm, 192 μm, 197 μm, 206 μm, 209 μm, 213 μm; most preferably 50 μm and 200 μm, for example 53 μm, 59 μm, 64 μm, 73 μm, 77 μm, 81 μm, 83 μm, 86 μm, 89 μm, 92 μm, 97 μm, 99 μm, 101 μm, 106 μm, 112 μm, 114 μm, 118 μm, 121 μm, 133 μm, 146 μm, 151 μm, 156 μm, 161 μm, 167 μm, 177 μm, 179 μm, 184 μm, 189 μm, 194 μm, 199 μm.

The dry powder formulation of the invention comprises vilanterol as an active agent. Vilanterol can be in the form of free base or a pharmaceutically acceptable salt, solvate, or physiologically functional derivative thereof, preferably in the form of a pharmaceutically acceptable salt, in the dry powder formulation. Pharmaceutically acceptable salts of vilanterol according to the invention include those formed with both organic and inorganic acids or bases. Pharmaceutical acceptable acid addition salts of vilanterol include those formed from hydrochloric, hydrobromic, sulphuric, citric, tartaric, phosphoric, lactic, pyruvic, acetic, trifluoroacetic, triphenylacetic, phenylacetic, substituted phenyl acetic e.g. methoxyphenyl acetic, sulphamic, sulphanilic, succinic, oxalic, fumaric, maleic, malic, glutamic, aspartic, oxaloacetic, methanesulphonic, ethanesulphonic, arylsulponic (for example p-toluenesulphonic, benzenesulphonic, naphthalenesulphonic or naphthalenedisulphonic), salicylic, glutaric, gluconic, tricarballylic, mandelic, cinnamic, substituted cinnamic (for example, methyl, methoxy, halo or phenyl substituted cinnamic, including 4-methyl and 4-methoxycinnamic acid and a-phenyl cinnamic acid), ascorbic, oleic, naphthoic, hydroxynaphthoic (for example 1-or 3-hydroxy-2-naphthoic), naphthaleneacrylic (for example naphthalene-2-acrylic), benzoic, 4-methoxybenzoic, 2or 4-hydroxybenzoic, 4-chlorobenzoic, 4-phenylbenzoic, bezeneacrylic (for example 1,4 benzenediacrylic) and isethionic acids. Pharmaceutical acceptable base salts of vilanterol include ammonium salts, alkali metal salts such as those of sodium and potassium, alkaline earth metal salts such as those of calcium and magnesium and salts with organic bases such as dicyclohexyl amine and N-methyl-D-glucamine.

In one embodiment of the present invention, vilanterol triphenylacetate is preferably used as active agent in the dry powder formulation.

Vilanterol is preferably present in an amount of 0.05 - 2.5%, more preferably present in an amount of 0.05 -1.5%, most preferably present in an amount of 0.1 -1.0 % by weight of the total formulation wherein the weight of vilanterol is calculated as the free base.

Drug deposition in the lung is mainly controlled by its mass median aerodynamic diameter. Particles larger than 5 μm are mostly trapped by oropharyngeal deposition and incapable of reaching the lungs while smaller than 1 μm are mostly exhaled without deposition. Particles with mass median aerodynamic diameters between 1 μm and 5 μm are expected to deposit in the lung periphery. Therefore, vilanterol with the mass median aerodynamic diameter between 1 μm and 5 μm is used as an active agent in the dry powder of the present invention.

As used herein, the terms "therapeutic agent", "active agent" and "drug" refer to a substance, as a chemical compound or complex, that has a measurable beneficial physiological effect on the body, such as a therapeutic effect in treatment and prophylaxis of a disease or disorder, when administered in an effective amount.

The phrase "effective amount" refers to that amount of a substance that produces some desired local or systemic effect at a reasonable benefit/risk ratio applicable to any treatment.

The term "mass median aerodynamic diameter" (MMAD) is a measure of the aerodynamic size of a dispersed

aerosol particle. The aerodynamic diameter is used to describe an aerosolized particle in terms of its settling behavior, and is the diameter of a unit density sphere having the same settling velocity, generally in air, as the particle in question. The aerodynamic diameter encompasses particle shape, density, and physical size. MMAD refers to the midpoint or median of the aerodynamic particle size distribution of an aerosolized collection of particles determined by Andersen cascade impactor (ACI), Next Generation Impactor (NGI), or Marple Miller impactor at each of the common flow rates.

Moisture uptake can directly affect the flowability of the powders and the force to detach the micronized particles from the carrier surface. Use for magnesium stearate in the formulation of the present invention, also helps to minimize the influence of penetrating moisture during the storage of said formulation and results in said formulation to be more stable against the moisture. Thus, the quality of the pharmaceutical formulation remains considerably better than conventional formulations which are free of magnesium stearate even on storage under extreme conditions of temperature and humidity. Therefore, use of magnesium stearate also improves the moisture resistance of the dry powder formulations.

However, magnesium stearate is poorly water soluble, its presence in such amount may rise some concerns as to a potential irritation or toxicity of this excipient, part of which can be inhaled by the patient together with the active ingredient. Therefore, it is important to determine the optimum concentration of the magnesium stearate that enables eliminating or minimizing potential irritation or toxicity of this excipient while getting balanced interparticulate forces between vilanterol particles and the carrier surface which will enable maximum aerosolisation deposition, and minimizing the influence of penetrating moisture during the storage of the formulation. According to the present invention, the optimum total amount of the magnesium stearate is found as less than 1.5% by weight based on the total amount of the dry powder formulation to achieve aforementioned effects at the same time. The preferred total amount of the magnesium stearate contained in the formulation of the present invention is between 0.02% and 1.0%, for example 0.03%, 0.04%, 0.05%, 0.06%, 0.07%, 0.08%, 0.09%, 0.15%, 0.25%, 0.35%, 0.45%, 0.55%, 0.65%, 0.75%, 0.85%, 0.95%; more preferred total amount of the magnesium stearate contained in the formulation of the present invention is between 0.05% and 0.75%, for example 0.065%, 0.075%, 0.085%, 0.095%, 0.12%, 0.14%, 0.16%, 0.18%, 0.20%, 0.22%, 0.24%, 0.26%, 0.28%, 0.30%, 0.32%, 0.34%, 0.36%, 0.38%, 0.40%, 0.42%, 0.44%, 0.46%, 0.48%, 0.52%, 0.54%, 0.56%, 0.58%, 0.60%, 0.62%, 0.64%, 0.66%, 0.68%, 0.70%, 0.72%, 0.74%; most preferred total amount of the magnesium stearate contained in the formulation of the present invention is between 0.10% and 0.50%, for example 0.11%, 0.13%, 0.17%, 0.19%, 0.21%, 0.23%, 0.27%, 0.29%, 0.31%, 0.33%, 0.37%, 0.39%, 0.41%, 0.43%, 0.47%, 0.49% by weight based on the total amount of the dry powder formulation.

According to present invention, the volume median diameter of the magnesium stearate is between 1 μm and 120 μm, for example 5 μm, 10 μm, 15 μm, 20 μm, 25 μm, 30 μm, 35 μm, 40 μm, 45 μm, 60 μm, 75 μm, 80 μm, 85 μm, 90 μm, 100 μm, 110 μm; preferably between 1 μm and 50 μm, for example 4 μm, 8 μm, 12 μm, 16 μm, 20 μm, 24 μm, 28 μm, 32 μm, 36 μm, 40 μm, 44 μm, 48 μm; more preferably between 1 μm and 25 μm, for example 3 μm, 6 μm, 9 μm, 11 μm, 14 μm, 18 μm, 21 μm, 23 μm; most preferably 1 μm and 15 μm, for example 2 μm, 4 μm, 7 μm, 8 μm; the volume median diameter of vilanterol or a pharmaceutically acceptable salt thereof, preferably vilanterol triphenylacetate, is between 0.5 μm and 6 μm, for example 0.6 μm, 0.8 μm, 1.2 μm, 1.4 μm, 1.6 μm, 1.8 μm, 2.0 μm, 2.2 μm, 2.4 μm, 2.6 μm, 2.8 μm, 3.0 μm, 3.2 μm, 3.4 μm, 3.6 μm, 3.8 μm, 4.0 μm, 4.2 μm, 4.4 μm, 4.6 μm, 4.8 μm, 5.0 μm, 5.2 μm, 5.4 μm, 5.6 μm, 5.8 μm; preferably 1 μm and 4 μm, for example 1.1 μm, 1.3 μm, 1.5 μm, 1.7 μm, 1.9 μm, 2.1 μm, 2.3 μm, 2.5 μm, 2.7 μm, 2.9 μm, 3.1 μm, 3.3 μm, 3.5 μm, 3.7 μm, 3.9 μm.

On the other hand, the pharmaceutically acceptable carrier may preferably consist of two fractions each of which has a different particle-size; fine carrier and coarse carrier. The type of the fine carrier can be same with or different from the type of the coarse carrier. The fine carrier and coarse carrier may constitute a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. According to present invention, lactose is preferably used as both of the fine carrier and coarse carrier. Therefore, lactose consists of fine lactose and coarse lactose each of which has a different particle-size. In one embodiment of the present invention, lactose is anyhdrous lactose or lactose monohydrate.

In dry powder formulations, it is known that the inclusion of fine carrier resulted in decreasing adhesion between the drug particles and carrier surface, therefore requiring a small amount of energy to detach the drug particles leading to an increased aerosol deposition performance. Because, the interactions between coarse and fine particles in the formation of mixtures which proposed that fine particles preferentially bind to areas of high energy (active sites) on the surface of the carrier, leading the drug particles to bind to areas with lower energy. Once aerosolised, the drug particles are more easily detached from the surface of the carrier, increasing the proportion of drug available for inhalation. Therefore, the carrier containing greater proportions of fine carrier give better aerosol deposition performance. Additionally, the particle size of the fine carrier is important as well as the amount of the the fine carrier for delivery performance of vilanterol in terms of fine particle fraction. According to present invention, the volume median diameter of the fine carrier particles, preferably fine lactose particles, is between 0.1 μm and 20 μm, for example 0.25 μm, 0.45 μm, 0.8 μm, 2 μm, 3 μm, 4 μm, 5 μm, 7 μm, 9 μm, 11 μm, 13 μm, 15 μm, 17 μm, 18 μm, 19 μm; preferably between 0.5 μm and 15 μm for example 0.75 μm, 1 μm, 1.5 μm, 2.5 μm, 3.5 μm, 4.5 μm, 5.5 μm, 6.5 μm, 7.5 μm, 8.5 μm, 9.5 μm,

10.5 μm, 11.5 μm, 12.5μ m, 13.5 μm, 14.5 μm; more preferably between 1 μm and 10 μm, for example 1.4 μm, 2.4 μm, 3.4 μm, 4.4 μm, 5.4 μm, 6.4 μm, 7.4 μm, 8.4 μm, 9.4 μm; most preferably 1 μm and 6 μm, for example 1.6 μm, 2.6 μm, 3.6 μm, 4.6 μm, 5.6 μm.

Additionally, if the fine carrier particles used in the dry powder formulation of the present invention, the amount of fine carrier particles also play a key role in drug deposition. The concentration of the fine carrier particles in the formulation has also an effect in the aerosolisation performance of the formulation. According to present invention, the amount of the fine carrier particles, preferably fine lactose particles, is less than 25%, preferably between 1% and 20, more preferably between 1% and 15%; most preferably between 1% and 10%, for example 2%, 3%, 4%, 5%, 6%, 7%, 8%, 9%, by weight based on the total amount of the dry powder formulation of the present invention.

The dry powder formulation of the present invention is used in the prophylaxis and treatment of clinical conditions for which a selective $\beta_2$-adrenoreceptor agonist is indicated. Such conditions include diseases associated with reversible airways obstruction such as asthma, chronic obstructive pulmonary diseases (COPD) (e. g. chronic and wheezy bronchitis, emphysema), respiratory tract infection and upper respiratory tract disease (e.g. rhinitis, including seasonal and allergic rhinitis).

According to the present invention, the dry powder formulations can be delivered by any suitable inhalation device that is adapted to administer a controlled amount of such a pharmaceutical formulation in dry powder form to a patient. Suitable inhalation devices may rely upon the aerosolisation energy of the patient's own breath to expel and disperse the dry powder dose. Alternatively, this energy may be provided by an energy source independent of the patient's inhalation effort, such as by impellers, patient/device created pressurised gas sources or physically (e. g. compressed gas) or chemically stored energy sources. Suitable inhalation devices can also be of the reservoir type i. e. where the dose is withdrawn from a storage vessel using a suitably designed dosing device or alternatively, inhalation devices that release drug from pre-metered units e. g. blisters, cartridges or capsules.

According to the present invention, the total amount of the dry powder formulation per unit-pack (blister, cartridge, capsule, etc) is between 3 mg and 25 mg, preferably 5 mg and 15 mg.

There are various various types of dry powder inhalers, for example, reservoir dry powder inhalers, unit-dose dry powder inhalers, pre-metered multi-dose dry powder inhalers, nasal inhalers or insufflators. The formulation of the invention may be presented in unit dosage form. Dry powder formulations for topical delivery to the lung by inhalation may, for example, be presented in capsules and cartridges of for example gelatin, or blisters of for example laminated aluminium foil, for use in an inhaler or insufflator. Each capsule, cartridge or blister may contain between 1 μg and 100 μg, preferably between 2 μg and 75 μg, more preferably 5 μg and 50 μg of vilanterol as free base.

According to the present invention, the dry powder formulation comprising vilanterol is in a for suitable for administration by oral and nasal inhalation.

Packaging of the formulation may be suitable for unit dose or multi-dose delivery. In one embodiment, the formulation suitable for inhaled administration may be incorporated into a plurality of sealed dose containers provided on medicament pack(s) (e.g. blister) mounted inside a suitable inhalation device. The containers may be rupturable, peelable or otherwise openable one-at-a-time and the doses of the dry powder composition administered by inhalation on a mouthpiece of the inhalation device, as known in the art. The medicament pack may take a number of different forms, for instance a disk-shape or an elongate strip. Representative inhalation devices are the Diskhaler® and Diskus® devices, marketed by GlaxoSmithKline. The Diskus® inhalation device is, for example, described in GB 2242134A. A dry powder inhalable composition, may also be provided as a bulk reservoir in an inhalation device, the device then being provided with a metering mechanism for metering a dose of the composition from the reservoir to an inhalation channel where the metered dose is able to be inhaled by a patient inhaling at a mouthpiece of the device. Exemplary marketed devices of this type are Turbuhaler® of AstraZeneca, Twisthaler® of Schering and Clickhaler® of Innovata.

A further delivery method for a dry powder inhalable composition is for metered doses of the composition to be provided in capsules (one dose per capsule) which are then loaded into an inhalation device, typically by the patient on demand. The device has means to rupture, pierce or otherwise open the capsule so that the dose is able to be entrained into the patient's lung when they inhale at the device mouthpiece. As marketed examples of such devices there may be mentioned Rotahaler® of GlaxoSmithKline and Handihaler® of Boehringer Ingelheim.

The dry powder pharmaceutical composition of vilanterol or a pharmaceutically acceptable salt thereof in accordance with this invention can be prepared using standard methods. Vilanterol or a pharmaceutically acceptable salt, pharmaceutically acceptable carrier and magnesium stearate can be sequentially or simultaneously mixed using any suitable blending apparatus, such as high shear mixer (for example a QMM, PMA or TRV series mixer) or a low shear tumbling mixer (a Turbula mixer). The particular components of the formulation can be admixed in any order. Pre-mixing of particular components may be found to be advantageous in certain circumstances. Before mixing process, if it is necessary, any of the component of the formulation can be micronized to obtain the component with the desired particle size. The progress of the mixing process can be monitored by carrying out content uniformity determinations. For example, the mixing apparatus may be stopped, materials removed using a sample thief and then analysed for homogeneity by High Performance Liquid Chromatography (HPLC).

The preferred process for the preparation of the dry powder formulation for inhalation of the present invention comprises:

a) the total amount of the pharmaceutically acceptable carrier is divided into five fractions and the first fraction of said carrier is put into a mixing vessel and it is mixed for a period of time to cover the inside of the wall of the mixing vessel,
b) then, the magnesium stearate and vilanterol or a pharmaceutically acceptable salt thereof are completely added onto the first fraction of the carrier in step a) and they are mixed for a period of time (Premix A),
c) second, third and fourth fraction of the carrier are added onto the Premix A respectively (Premix B); after every addition of one fraction of the carrier to the mixture, they are mixed for a period of time,
d) finally, last fraction of the carrier is added onto the Premix B and they are mixed for a period of time to obtain the dry powder formulation of the invention.

In one embodiment of the present invention, vilanterol triphenylacetate salt is used for the preparation of the dry powder formulation.

In another embodiment of the present invention, the pharmaceutically acceptable carrier used in the preparation of the dry powder formulation is selected from the group comprising lactose, mannitol, glucose, trehalose, cellobiose, sorbitol, maltitol or a combination of two or more of them, for example a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. Lactose is preferably used as the pharmaceutically acceptable carrier. Lactose used for the preparation of the dry powder formulation is anyhdrous lactose or lactose monohydrate.

Therefore, the more preferred process for the preparation of the dry powder formulation for inhalation comprising:

a) the total amount of the lactose is divided into five fractions and the first fraction of the lactose is put into a mixing vessel and it is mixed for a period of time to cover the inside of the wall of the mixing vessel,
b) then, the magnesium stearate and vilanterol triphenylacetate are completely added onto the first fraction of the lactose in step a) and they are mixed for a period of time (Premix A),
c) second, third and fourth fraction of the lactose are added onto the Premix A respectively (Premix B); after every addition of one fraction of the lactose to the mixture, they are mixed for a period of time,
d) finally, last fraction of the lactose is added onto the Premix B and they are mixed for a period of time to obtain the dry powder formulation of the invention.

In one embodiment of the present invention, in the preparation of the dry powder formulation, the Premix B is preferably sieved after step c). Then, the last fraction of the lactose is preferably added, through the same sieve that is used to sieve the Premix B, onto the Premix B to remove the remains attached to the sieve.

According to the present invention, the pharmaceutically acceptable carrier, preferably lactose, can be divided into five equal fractions or into five fractions in any proportion.

If the coarser lactose and the fine lactose is used for the preparation of the present invention, the coarser lactose is divided into five equal fractions or into five fractions in any proportion in step a), whereas, in step b), the fine lactose is added together with magnesium stearate and vilanterol triphenylacetate onto the first fraction of the coarse lactose.

According to the present invention, vilanterol or a pharmaceutically acceptable salt thereof can be used in combination with one or more other therapeutic agents that is selected from a group comprising anti-inflammatory agents, anticholinergic agents (particularly a muscarinic ($M_1$, $M_2$, or $M_3$) receptor antagonist), other $\beta_2$-adrenoreceptor agonists, antiinfective agents (e.g. antibiotics, antivirals), or antihistamines for the preparation of the dry powder formulation. The invention thus provides, in a further embodiment, a combination comprising vilanterol or a pharmaceutically acceptable salt, solvate or physiologically functional derivative thereof, preferably vilanterol triphenylacetate, together with one or more other therapeutical active agents that is selected from a group comprising an anti-inflammatory agent (e.g. a corticosteroid or an NSAID), an anticholinergic agent, another $\beta_2$- adrenoreceptor agonist, an antiinfective agent (e. g. an antibiotic or an antiviral), or an antihistamine. Preferred are combinations comprising vilanterol or a pharmaceutically acceptable salt, solvate or physiologically functional derivative thereof, preferably vilanterol triphenylacetate, together with a corticosteroid (such as mometasone, fluticasone, budesonide) and/or an anticholinergic (such as tiotropium, oxitropium, glycopyrronium, ipratropium, aclidinium) and/or a PDE-4 inhibitor (such as roflumilast, rolipram, ibudilast, cilomilast) for the preparation of the dry powder formulation.

The other therapeutic ingredient (s) may be used in the form of salts, (e. g. as alkali metal or amine salts or as acid addition salts), or prodrugs, or as esters (e. g. lower alkyl esters), or as solvates (e. g. hydrates). It will be clear also that where appropriate, the therapeutic ingredients may be used in optically pure form.

Vilanterol or a pharmaceutically acceptable salt thereof, preferably vilanterol triphenylacetate, and one or more other therapeutic agent(s) may be administered separately, sequentially or simultaneously in separate or combined pharmaceutical formulations in dry powder form. Vilanterol or a pharmaceutically acceptable salt thereof, preferably vilanterol triphenylacetate, and said other therapeutic agent(s) may be formulated separately and presented in separate packs or devices, or said individually formulated components may be present in a single pack or device. Where appropriate, the individual therapeutic agents may be admixed within the same formulation, and presented as a fixed pharmaceutical combination or formulated as a seperate formulation including pharmaceutically acceptable carriers and/or additives.

In one embodiment, vilanterol or a pharmaceutically acceptable salt thereof, preferably vilanterol triphenylacetate, may be used in combination with 6a, 9a-difluoro- 17a-[(2-furanylcarbonyl)oxy]-11β-hydroxy-16a-methyl-3-oxo-androsta-1,4-diene-17β-carbothioic acid S-fluoromethyl ester (fluticasone furoate) for the preparation of a pharmaceutical product for the treatment and prophylaxis of inflammatory or respiratory tract diseases.

Fluticasone furoate has also been the subject of extensive studies in animal models and humans and has been found to be a long acting inhaled glucocorticosteroid which has potential for once-daily administration to the lungs.

Fluticasone furoate is considered to have potential in the treatment of respiratory tract disease such as chronic obstructive pulmonary disease, chronic bronchitis, asthma, chronic respiratory obstruction, pulmonary fibrosis, pulmonary emphysema and allergic rhinitis.

For use according to the present invention, fluticasone furoate may be administered by inhalation at a dose of from about 25 μm to about 750 μm daily, and if necessary in divided doses. Thus, the daily dose of vilanterol may be at a dose of from about 1 μm to about 100 μm daily as free base, and if necessary in divided doses. In general, fluticasone furoate will be administered as a once-daily dose. Fluticasone furoate may be provided in unit dose form, for example as described for vilanterol. Each unit dose of fluticasone furoate may contain between 25 μm and 750 μm of fluticasone furoate. The active agents (or therapeutic agents or drugs) used in the dry powder formulation of the invention preferably have a mass median aerodynamic diameter between 1 μm and 5 μm.

In another embodiment of the present invention, the present invention provides a pharmaceutical product comprising the dry powder formulation for inhalation of the present invention in combination with a dry powder formulation for inhalation comprising one or more other therapeutic agent(s), preferably 6a, 9a-difluoro- 17a-[(2-furanylcarbonyl)oxy]-11β-hydroxy-16a-methyl-3-oxo-androsta-1,4-diene-17β-carbothioic acid S-fluoromethyl ester (fluticasone furoate) and pharmaceutically acceptable carrier and/or additive.

In one embodiment said combination may be presented in the form of a pack comprising a dry powder formulation for inhalation comprising vilanterol or a pharmaceutically acceptable salt thereof, preferably vilanterol triphenylacetate, of the present invention and a separate dry powder formulation for inhalation of 6a, 9a-difluoro- 17a-[(2-furanylcarbonyl)oxy]-11β-hydroxy-16a-methyl-3-oxo-androsta-1,4-diene-17β-carbothioic acid S-fluoromethyl ester (fluticasone furoate).

Said pack may comprise two separate inhaler devices, containing respectively the separate formulations of vilanterol or a pharmaceutically acceptable salt thereof, preferably vilanterol triphenylacetate, and fluticasone furoate. Inhaler devices for delivery of fluticasone furoate include those described hereinabove for delivery of vilanterol.

Said pack may also comprise a delivery device which permits separate containment of the dry powder formulation of vilanterol or a pharmaceutically acceptable salt thereof, preferably vilanterol triphenylacetate, and the dry powder formulation of fluticasone furoate. Thus, for example, the individual compounds of the combination are administrable simultaneously but are stored separately, e.g. in separate pharmaceutical compositions.

In one embodiment, a delivery device permitting separate containment of active agents is an inhaler device having two medicament packs in peelable blister strip form, each pack containing pre-metered doses in blister pockets arranged along its length. Said device has an internal indexing mechanism which, each time the device is actuated, peels opens a pocket of each strip and positions the packs so that each newly exposed dose of each pack is adjacent a manifold which communicates with a mouthpiece of the device. When the patient inhales at the mouthpiece, each dose is simultaneously drawn out of its associated pocket into the manifold and entrained via the mouthpiece into the patient's respiratory tract. Thus, each time the device is used, the patient is administered a combination therapy consisting of a dose from each medicament pack.

The present invention further provides a pharmaceutical product comprising a combination of vilanterol or a pharmaceutically acceptable salt thereof, preferably vilanterol triphenylacetate, and fluticasone propionate wherein at least vilanterol or a pharmaceutically acceptable salt thereof is formulated with magnesium stearate. Fluticasone furoate is also formulated with carrier, preferably fine and coarse carrier, optionally with magnesium stearate.

In another embodiment of the present invention, the present invention provides a pharmaceutical product comprising as separate formulation:

(a) a dry powder formulation comprising vilanterol or a pharmaceutically acceptable salt thereof of the present invention; and

(b) a dry powder formulation for inhalation comprising 6a, 9a-difluoro- 17a-[(2-furanylcarbonyl)oxy]-11β-hydroxy-16a-methyl-3-oxo-androsta-1,4-diene-17β-carbothioic acid S-fluoromethyl ester (fluticasone furoate), pharmaceutically acceptable carrier and/ or additive.

Vilanterol triphenylacetate salt is preferably used as the active agent in the dry powder formulation (a) of above mentioned pharmaceutical product.

According to the present invention, the pharmaceutically acceptable carrier used in the dry powder formulation of fluticasone furoat (b) is selected from the group comprising lactose, mannitol, glucose, trehalose, cellobiose, sorbitol, maltitol or a combination of two or more of them, for example a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. Lactose is preferably used as a pharmaceutically acceptable carrier in the dry powder formulation of fluticasone furoat. In one embodiment of the present invention, lactose is anyhdrous lactose or lactose monohydrate. On the other hand, if the dry powder formulation of fluticasone furoat comprises an additive, the additive is selected from a group comprising magnesium stearate, stearic acid, sodium lauryl sulphate, sodium stearyl fumarate, stearyl alcohol and sodium benzoate. Preferably, the magnesium stearate is used as the additive in said formulation.

Another embodiment of the invention is a method for the treatment and prophylaxis of inflammatory or respiratory tract diseases, which method comprises administering either sequentially or simultaneously, to a patient in need thereof, a pharmaceutical product comprising the dry powder formulation of vilanterol or a pharmaceutically acceptable salt thereof, preferably vilanterol triphenylacetate, and fluticasone furoate as defined above.

In one embodiment of the invention the inflammatory or respiratory disease is selected from the group consisting of chronic obstructive pulmonary disease, chronic bronchitis, asthma, chronic respiratory obstruction, pulmonary fibrosis, pulmonary emphysema and allergic rhinitis.

In another embodiment of the invention the pharmaceutical product may be used for the treatment of respiratory disease, and more specifically the treatment of asthma and/or chronic obstructive pulmonary disease (COPD), by simultaneous or successive administration of vilanterol or a pharmaceutically acceptable salt thereof and fluticasone furoate.

The present invention is explained in detail in the following examples. These examples are not limiting the scope of the present invention and are to be considered under the light of the foregoing detailed disclosure.

**Example**

| Formulation No | Vilanterol (as free base) | Lactose | $X=Wv/W_{Mg}$ | $Y=VMD_v/VMD_{MgSt}$ | X/Y | Total amount |
|---|---|---|---|---|---|---|
| 1 | 0.10%-1.5% | 85%-99% | 0.2 | 0.5 | 1:2.5 | 3 - 25 mg |
| 2 | 0.10%-1.5% | 85%-99% | 0.1 | 0.3 | 1:3 | 3 - 25 mg |
| 3 | 0.10%-1.5% | 85%-99% | 0.8 | 0.05 | 16:1 | 3 - 25 mg |
| 4 | 0.10%-1.5% | 85%-99% | 0.2 | 4 | 1:20 | 3 - 25 mg |
| 5 | 0.10%-1.5% | 85%-99% | 0.4 | 0.5 | 1:1.25 | 3 - 25 mg |

*In the table,* **Wv** *is abbreviation of the total amount of vilanterol whereas* $\mathbf{W_{Mg}}$ *is abbreviation of the total amount of magnesium stearate.* $VMD_V$ *is abbreviation of the volume median diameter of vilanterol whereas* $VMD_{MgSt}$ *is abbreviation of the volume median diameter of the magnesium stearate. The percentage amount range of each component (showed in the table) is calculated based on the total amount of the total weight of the formulation.*

**Preparation of Formulation-I**

For the preparation of Formulation-1 in the table, initially the components of the Formulation-1 is weighted to the amount falling within the range that is showed in the table for each component. If it is necessary, any of the components of the formulation is micronized in a microniser (e.g. air-jet mill micronizer) to obtained said component with desired volume median diameter defined in the description before the mixing process. For the preparation of the Formulation-1, the total amount of the lactose is divided into five fractions and the first fraction of the lactose is put into a mixing vessel and it is mixed for a period of time to cover the inside of the wall of the mixing vessel. Then, the magnesium

stearate and vilanterol triphenylacetate are completely added onto the first fraction of the lactose in step a) and they are mixed for a period of time (Premix A). Second, third and fourth fraction of the lactose are added onto the Premix A respectively (Premix B); after every addition of one fraction of the lactose to the mixture, they are mixed for a period of time. Finally, last fraction of the lactose is added onto the Premix B and they are mixed for a period of time to obtain the dry powder formulation (Formulation-1). Each of the mixing processes during the preparation of the dry powder formulation is performed using a high shear mixer or a low shear tumbling mixer whichever is appropriate. The obtained Formulation-1 is the dry powder formulation wherein the quotient X is the ratio of the total amount of vilanterol (as free base) to the total amount of the magnesium stearate and the quotient Y is the ratio of the volume median diameter of vilanterol triphenylacetate to the volume median diameter of the magnesium stearate, in which the ratio of the quotient X to the quotient Y is 1:2.5.

Formula-2, Formula-3, Formula-4 and Formula-5 given in the table were prepared using the above-described procedure for the preparation of Formula-1.

## Claims

**1.** A dry powder formulation for inhalation comprising:

- vilanterol or a pharmaceutically acceptable salt thereof,
- a pharmaceutically acceptable carrier, and
- magnesium stearat,

wherein the quotient X is the ratio of the total amount of vilanterol (as free base) to the total amount of the magnesium stearate and the quotient Y is the ratio of the volume median diameter of vilanterol or a pharmaceutically acceptable salt thereof to the volume median diameter of the magnesium stearate, in which the ratio of the quotient X to the quotient Y is between 100:1 and 1:100.

**2.** The dry powder formulation for inhalation according to claim 1, wherein the pharmaceutically acceptable salt of vilanterol is triphenylacetate salt.

**3.** The dry powder formulation for inhalation according to claim 1, wherein the pharmaceutically acceptable carrier is selected from the group comprising lactose, mannitol, glucose, trehalose, cellobiose, sorbitol, maltitol or a combination of two or more of them.

**4.** The dry powder formulation for inhalation according to claim 3, wherein lactose is used as the pharmaceutically acceptable carrier.

**5.** The dry powder formulation for inhalation according to claim 4, wherein the volume median diameter of the lactose is between 30 μm and 250 μm.

**6.** The dry powder formulation for inhalation according to claim 1, wherein the amount of the magnesium stearate is less than 1.5% by weight based on the total amount of the dry powder formulation.

**7.** The dry powder formulation for inhalation according to any one of the preceding claims, wherein the lactose consists of fine lactose and coarse lactose each of which has a different particle-size.

**8.** A process for the preparation of the dry powder formulation for inhalation according to any one of the preceding claims comprising:

a. the total amount of the lactose is divided into five fractions and the first fraction of the lactose is put into a mixing vessel and it is mixed for a period of time to cover the inside of the wall of the mixing vessel,
b. then, the magnesium stearate and vilanterol triphenylacetate are completely added onto the first fraction of the lactose in step a) and they are mixed for a period of time (Premix A),
c. second, third and fourth fraction of the lactose are added onto the Premix A respectively (Premix B); after every addition of one fraction of the lactose to the mixture, they are mixed for a period of time,
d. finally, last fraction of the lactose is added onto the Premix B and they are mixed for a period of time to obtain the dry powder formulation.

**9.** The dry powder formulation for inhalation according to any one of the claims 1 to 7, wherein vilanterol or a pharmaceutically acceptable salt is used in combination with one or more other therapeutic agents that is selected from a group comprising an anti-inflammatory agent (e.g. a corticosteroid or an NSAID), an anticholinergic agent, another β2- adrenoreceptor agonist, an antiinfective agent (e. g. an antibiotic or an antiviral), or an antihistamine for the preparation of the dry powder formulation.

**10.** The dry powder formulation for inhalation according to claim 9, wherein vilanterol or a pharmaceutically acceptable salt and one or more other therapeutic agent(s) are administered separately, sequentially or simultaneously in separate or combined pharmaceutical formulations in dry powder form.

**11.** A pharmaceutical product comprising:

- the dry powder formulation for inhalation according to any one of the claims 1 to 7,
- a dry powder formulation for inhalation comprising 6a, 9a-difluoro- 17a-[(2-furanylcarbonyl)oxy]-11β-hydroxy-16a-methyl-3-oxo-androsta-1,4-diene-17β-carbothioic acid S-fluoromethyl ester (fluticasone furoate) and pharmaceutically acceptable carrier and/or additive.

Europäisches Patentamt
European Patent Office
Office européen des brevets

# EUROPEAN SEARCH REPORT

Application Number
EP 15 17 2162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/097115 A1 (GLAXO GROUP LTD [GB]; BAKER DARRELL [GB]; BRUCE MARK [GB]; THOMAS MARI) 2 September 2010 (2010-09-02) * page 1, line 20 - page 2, line 10; page 3, lines 2-10; page 6, line 23 - page 7, line 19 * ----- | 1-11 | INV. C07C31/135 A61K31/40 A61P11/08 |

TECHNICAL FIELDS SEARCHED (IPC)
C07C
A61K
A61P

The present search report has been drawn up for all claims

1

| Place of search | Date of completion of the search | Examiner |
|---|---|---|
| Munich | 30 September 2015 | Schmid, Arnold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 2162

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2015

| Patent document cited in search report | Publication date | Patent family member(s) | Publication date |
|---|---|---|---|
| WO 2010097115 A1 | 02-09-2010 | EP 2400950 A1 | 04-01-2012 |
| | | JP 2012518663 A | 16-08-2012 |
| | | US 2011319371 A1 | 29-12-2011 |
| | | WO 2010097115 A1 | 02-09-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03024439 A1
- GB 2242134 A